# EUROPEAN PATENT SPECIFICATION

(11) **EP 2 614 810 B1**
(45) Date of publication and mention of the grant of the patent: **06.12.2017**
(21) Application number: 13151271.7
(22) Date of filing: 15.01.2013
(51) Int. Cl.: A61K 6/00, A61C 1/00, A61C 3/00, A61C 7/02, A61C 9/00, A61C 13/12, C08L 27/00, C08L 33/10, C08L 67/00, C08L 69/00, C08L 75/04, C08L 77/00, C08L 81/00, C08L 101/06

(54) **Thermoplastic-based polymer adhesive compositions and apparatuses for their use in dental applications**
Auf Thermoplast basierte Polymerhaftzusammensetzungen und Vorrichtungen für ihre Verwendung in Zahnanwendungen
Compositions adhésives de polymère à base de thermoplastique et appareils pour leur utilisation dans des applications dentaires

(30) Priority: 16.01.2012 US 201261586980 P
(43) Date of publication of application: 17.07.2013
(73) Proprietor: Kerr Corporation, Orange, CA 92857 (US)
(72) Inventor: Krikorian, Vahik, La Canada, California 91011 (US); Lobsenz, James, Redondo Beach, California 90278 (US); Durali, Mehdi, Carlsbad, California 92009 (US); Wong, Raymond F, Chino Hills, California 91709 (US); Chen, Xiangxu, Diamond Bar, California 91765 (US)
(74) Representative: Findlay, Alice Rosemary

(56) References cited:
- EP-A2- 2 022 464
- US-A1- 2007 232 718
- DATABASE CAPLUS [Online] CHEMICAL ABSTRACTS SERVICE, COLUMBUS, OHIO, US; 4 June 1998 (1998-06-04), XP002710413, retrieved from STN Database accession no. 1998:335482 -& JP H10 139613 A (KURARAY CO; JAPAN INST ADVANCED DENTISTRY) 26 May 1998 (1998-05-26)
- DATABASE CAPLUS [Online] CHEMICAL ABSTRACTS SERVICE, COLUMBUS, OHIO, US; 1 July 1992 (1992-07-01), XP002710414, Database accession no. 1992:578371 -& JP H04 187609 A (NIKKISO CO LTD) 6 July 1992 (1992-07-06)

## Description

The present invention relates generally to thermoplastic-based polymer adhesives for use in dentistry and apparatuses for using those adhesives.

The dental clinician depends on adhesives and cements for completing various dental treatments. For example, dental treatment may require installation of an indirect restoration so that the patient may regain full utilization of one or more teeth. Unlike direct restorations, which are formed in the mouth, indirect restorations are constructed external to the patient. Indirect restorations may include inlays, onlays, crowns, veneers, and bridges. Once constructed, usually with the aid of a dental impression, the clinician must install the indirect restoration. The restoration is secured to a portion of the patient's tooth with a dental adhesive or cement. While removable or temporary restorations are available, for example, dentures, many indirect restorations require a permanent bond to the tooth.

Dental cements are also used in orthodontic treatment. Orthodontic treatment often involves attaching an appliance to the tooth to which applied forces are used to move the tooth. As such, orthodontic appliances represent a principal component of corrective orthodontic treatment devoted to improving a patient's dentition. Orthodontic appliances may include brackets or other devices. Using the orthodontic bracket as an example, an orthodontist may affix orthodontic brackets to the patient's teeth with an adhesive and engage an archwire (another type of orthodontic appliance) into a slot of each bracket. The archwire applies corrective forces that coerce the teeth to move into orthodontically correct positions. The adhesive used is critical to the realization of the full benefit of the orthodontic bracket. Like brackets, many other appliances also rely on an adhesive to provide their desired function.

Accordingly, a large variety of dental cements are designed to bond restorations or orthodontic appliances to a tooth for treatment. To this end, dental cements include various resin-based adhesives that undergo a "curing" process whereby the adhesive hardens and bonds to both the tooth material and the restoration or appliance. The curing process is generally a one-way reaction of components within the cement. For example, curing may include enabling cross-linking of the polymers in the cement. Generally the curing process is non-reversible. Therefore, while most of these cements are pliable so as to allow their application onto the appropriate surfaces, the curing process hardens the cement to make it permanent. Because the reaction is non-reversible, the hardness and the integrity of the cement may not be easily or conveniently reversed either.

This one-way or non-reversibility creates problems in dental treatment, particularly with indirect restorations and appliances, for example, because the accuracy of placement of the restoration or the appliance may be a significant factor in whether the treatment is successful. In this regard, should the clinician misplace the restoration or appliance, that misplacement may not be easily correctable without causing undo patient discomfort or without damaging the patient's tooth. In addition, should a restoration or appliance crack or fail some months or years after placement, removal of the faulty component may not be a simple process. Rather, the permanence of the cement may make the replacement of the damaged or faulty components difficult. In addition, in cases in which a temporary restoration is necessary, removal of the restoration secured with the cement becomes problematic.

There are other drawbacks to these types of cements. Generally, improper application or curing of the cement may result in premature debonding of the restoration or appliance from the tooth. For example, insufficient curing or premature curing of the cement may leave the mechanical and chemical stability of the cement insufficient to withstand long-term exposure in the oral environment. Thus, an orthodontic bracket or restoration may eventually debond from the tooth during treatment. This unintentional interruption in treatment requires the attention of a clinician for repair and often necessitates a special trip to the doctor's office.

Still other drawbacks include cracking during curing. Again, this may facilitate failure of the bond during treatment. And, other drawbacks may include the application process itself. Curable polymers are typically viscous liquid-like compounds. Depending on the viscosity, the polymer may be difficult to apply or the liquid-like compound may run off the surface to which it is applied. In either case, the low viscosity liquid-like nature of the curable polymers may present application issues for the clinician.

Alternative adhesives include thermoplastic polymers, which may be heated prior to or during application of the orthodontic appliance. There are, however, drawbacks and limitations to the use of these materials in dentistry and to treatment methods using these materials as well.

EP2022464 discloses a method for temporarily widening a gingival sulcus. A composition comprising a polymerizable monomer having at least one ethylenically unsaturated group, a photo polymerization initiator, and a fine inorganic powder is inserted within a gingival sulcus to be widened, and the composition is thereafter irradiated in situ to polymerize the composition. The result is rubbery material that is easily removable from the widened sulcus.

The invention provides a device for use in a gingival retraction procedure to enlarge a gingival sulcus, the device characterised in that it comprises a thermoplastic-based polymer adhesive comprising a thermoplastic polymer, and an additional polymer or additive including at least one moiety, monomer, or prepolymer capable of changing at least one property of the thermoplastic-based polymer adhesive when exposed to an activation energy, and a tubular wall or ring structure configured to fit around a tooth and including the thermoplastic-based polymer adhesive, the structure being configured to be operatively coupled to a source of energy that is capable of applying energy to the thermoplastic-based polymer adhesive to heat the thermoplastic-based polymer adhesive before the thermoplastic-based polymer adhesive is inserted into the sulcus.

In one embodiment, the activation energy is heat energy and the additional polymer or additive is activated at a predetermined temperature. In one embodiment, the activation energy is sonic or ultrasonic energy. In one embodiment, the additional polymer or additive is contained within a capsule that is configured to release the additional polymer or additive upon application of the activation energy. In one embodiment, the thermoplastic polymer is selected from the group consisting of polyamides, polyaryl ketones, polyesters, polyimides, polysulfones, polyurethanes, polycarbonates, polyolefines, halogenated polyolefines, polyacetals, polyacrylates, polymethacrylates, and polyphenylene sulfides. In one embodiment, the additional polymer or additive is bisphenol A glycidyl methacrylate (BisGMA), urethane dimethacrylate, triethylene glycol dimethacrylate, or a derivative thereof. In one embodiment, the additional polymer or additive is configured to be activated by sonic or ultrasonic energy.

In one embodiment, the tubular wall or ring structure defines channels that contain the thermoplastic-based polymer adhesive. The channels are configured to be in communication with a gingiva when the structure is placed around the tooth. The thermoplastic-based polymer adhesive is movable within the channels toward the sulcus.

In one embodiment, a portion of the tubular wall or ring structure is configured for contact with a gingiva and is formed of the thermoplastic-based polymer adhesive.

The invention will now be further described by way of example with reference to the accompanying drawings, in which:
Fig. 1 is an elevation view of an embodiment illustrating a dual-layer sheet;
Fig. 2 is an elevation view of the embodiment shown in Fig. 1 in an installed position;
Fig. 3 is a cross-sectional view of one embodiment of a layer including a plurality of capsules;
Fig. 4 is an elevation view of a retraction device according to an embodiment of the invention;
Fig. 5 is an elevation view of an alternative embodiment of a retraction device shown in Fig. 4.
Fig. 6 is an elevation view illustrating one embodiment of a device for removal and/or reattachment of an orthodontic appliance;
Fig. 7 is an elevation view illustrating an alternative embodiment of the device shown in Fig. 6;
Fig. 8 is an elevation view illustrating one embodiment of a device for removal and/or reattachment of an orthodontic appliance;
Fig. 9 is an elevation view illustrating another embodiment of a device for removal and/or reattachment of an orthodontic appliance; and
Fig. 10 is a perspective view of a self-ligating orthodontic bracket.

A thermoplastic-based polymer adhesive as disclosed herein may be used to reversibly secure a dental component to a tooth. As used herein, a dental component may refer to components used in restorative, orthodontic, implant, and/or endodontic dentistry. By way of example, dental components used in restorative dentistry include indirect restorations, such as, but not limited to, inlays, onlays, veneers, and bridges. Orthodontic appliances include, but are not limited to, orthodontic brackets. As will be described in more detail below, the thermoplastic-based polymer adhesive has reversible properties. The reversible nature of the adhesive may be tuned or adjusted so that the level of reversibility is controllable. In addition, the properties of the adhesive may become gradually or abruptly irreversible when subject to an activation energy of a predetermined level.

To those and other ends, the thermoplastic-based polymer adhesive includes at least one thermoplastic polymer. Thermoplastic polymers are those polymers capable of being softened or melted when heated and then hardened or crystallized by subsequent cooling from the heated state and may, when heated according to some embodiments of the invention, undergo a substantially physical rather than chemical change. Generally, this viscosity-temperature relationship is reversible such that a thermoplastic polymer may be repeatedly heated and then cooled with the viscosity of the polymer changing with temperature according to the same known relationship. Thermoplastic polymers of the adhesive may be highly crystalline, semicrystalline, or completely amorphous. Exemplary thermoplastic polymers may include, but are not limited to polyamides, polyaryl ketones, polyesters, polyimides, polysulfones, polyurethanes, polycarbonates, polyolefines (especially halogenated polyolefines), polyacetals, polyacrylates (including methacrylates), and polyphenylene sulfides. Blends and copolymers of such polymers can also be used. In addition, polymers, such as, polyacrylonitrile, cellulose acetate, ethylene vinyl acetate, ethylene vinyl alcohol, polybutylene, poly ether ether ketone, poly phenylene oxide, and poly phenylene sulfide may be used.

The thermoplastic-based polymer adhesive includes at least one additional polymer or additive. While the thermoplastic-based polymer adhesive includes a thermoplastic polymer that generally does not undergo a chemical change, the additional polymers or additives may facilitate desired chemical changes, as is described below.

In this regard, the additional polymers or additives may be part of the polymeric backbone or react with the thermoplastic polymer. This reaction may produce a chemical change in the adhesive. However, the timing of the reaction or chemical change is controllable by the clinician. Activating the adhesive may include exposing the adhesive to an activation energy of a predetermined threshold level and/or for a predetermined amount of time, for example, to heat the adhesive to a predetermined activation temperature. When the adhesive is at a temperature below the activation temperature, the additional polymers or additives may not significantly inhibit the reversible properties of the thermoplastic-based polymer adhesive.

In this sense, the thermoplastic-based polymer adhesive maintains at least some reversibility in at least one property prior to activation. For example, where heating the adhesive to the predetermined activation temperature is required to activate the additional polymer or additive, viscosity of the adhesive may be reversible in the temperature range below the activation temperature. Thus, the activation energy may be used to heat the adhesive in the range of temperatures that result in reversible changes up to the activation temperature without activating the adhesive. For example, the clinicians may then completely reverse the viscosity of the adhesive at least once or repeatedly prior to activation of the adhesive with the viscosity response to heating and cooling being similar during each heating/cooling cycle. Advantageously, the clinician may then remove and reattach the dental component to the tooth at temperatures at or below the predetermined activation temperature. Thus, the clinician may correct an initial misplacement of the dental component at least once.

Once the clinician has placed the dental component at the correct position, chemical changes may be induced by activating the additional polymer or additive. The properties of the adhesive may become at least partially irreversible or non-reversible once activated. As set forth above, in one embodiment, activating the adhesive includes heating the adhesive with the activation energy to the predetermined temperature at or above which the properties of the adhesive change. The resulting change in the adhesive may not be reversible. For example, following activation of the additional polymer or additive, the viscosity-temperature relationship may be completely eliminated. That is, the adhesive, after activation, may exhibit little if any change in viscosity with a change in temperature. It will be appreciated, however, that rather than a complete elimination of the reversibility, the reversibility may be fully or at least partially maintained.

In general, activation may include selectively exposing the adhesive to a source of the activation energy. The activation energy or trigger may include ultrasonic energy or another energy source, such as, electromagnetic radiation (e.g., microwaves) or electrical energy to heat the composition to above the activation temperature or may include a change in environmental chemistry, such as, but not limited to a change in pH or a change in the ionic strength of the surrounding environment to above or below a certain predetermined value. By way of example, the source of the activation energy may include a transducer capable of producing sonic or ultrasonic energy having a frequency in the range of from about 2 kHz to about 200 kHz, and by way of further example, the frequency may be in the range of from about 20 kHz to about 80 kHz. The ultrasonic energy may have oscillation amplitude of from about 1 µm to about 200 µm with a power of from about 2 to about 20 W/mm² of targeted surface.

With regard to the additional polymers or additives, these may include one or more other polymers that may not exhibit thermoplastic properties. The thermoplastic-based polymer adhesive may, therefore, be a composite of thermoplastic polymers and non-thermoplastic polymers. In one embodiment, the additional polymers or additives may include, for example, thermosetting polymers. Thus, the adhesive may be a composite of thermoplastic and thermosetting polymers. In this case, following activation, the adhesive may undergo a setting or curing-type reaction in which the thermosetting polymers alter or transform the viscosity-temperature relationship of the adhesive so that it is no longer fully reversible.

The type, quantity, and method of introducing the additional polymers and additives to the adhesive is controlled so that the reversibility of the thermoplastic-based polymer adhesive is maintained or changes little prior to activation of the adhesive, as set forth above. In this regard, the additional polymer or additive may include specific moieties that are formulated or mixed with the thermoplastic polymer to change the properties of the adhesive upon application of the activation energy. The type and concentration of moiety may be selected to facilitate a specific response in or a change to the structure of the thermoplastic polymer.

In one embodiment, the additional polymer or additive may include a moiety with a resin-based chemistry, such as, a thermoset-like chemistry, that may be self-curable or curable by application of light. Other suitable reactive polymers or additives may include an initiator or catalyst that activates an existing functional group on the thermoplastic polymer backbone or that activates a second component of the thermoplastic polymer composition that is capable of further polymerization and crosslinking. By way of example, the reactive polymer or additives may include bisphenol A glycidyl methacrylate (BisGMA), urethane dimethacrylate, triethylene glycol dimethacrylate, and/or derivatives thereof.

In one embodiment, the additional polymers or additives may include a moiety that is selected because of its affinity for the chains in the thermoplastic polymer. For example, once activated, the moiety may interact with the adjacent chains of the thermoplastic polymer by Van der Waals forces, by hydrogen bonding, or by stronger chemical bonding to change the properties of the adhesive in a predefined manner. In one embodiment, one or more moieties are added by grafting or by random copolymerization to the backbone of the thermoplastic polymer. One or more of the moieties may then be activated by exposing the copolymer in the adhesive to a predetermined level of the activation energy. In one embodiment, the moieties may be bi-functional and may form a chemical and/or physical crosslinking when activated.

In one embodiment, the concentration of the moiety is in an amount sufficient to produce the desired change, for instance, the concentration may determine the extent or level of the change in the reversibility of the adhesive. By way of example, the amount of the moiety may be only a few hundredths of a percent, for example, about 0.01 wt. % to greater amounts. The concentration may, however, require substantially greater concentrations, for example, up to about 90 wt. %.

In addition to or as an alternative to the moieties described above, the additional polymer or additive may include a monomer containing unsaturated bonds that is copolymerized with the thermoplastic polymer. The monomer may then be activated at a specific time to participate in a cross-linking reaction. By way of example, suitable monomers include, but are not limited to, diacrylates; dimethacrylates; and dienes, such as, butadiene, isoprene, and chloroprene. Additionally, a moiety capable of cross-linking may exist as a free prepolymer that is activated by one of the activation energies as desired. Exemplary prepolymers include but are not limited to 2,2-bis[4(2-hydroxy-3-methacryloxy-propyloxy)-phenyl] propane (Bis-GMA), urethane dimethacrylate (UDMA), and triethylene glycol dimethacrylates (TEGDMS). In addition or as an alternative, the monomer may contain a functional moiety that may exist as a random, block, or alternative copolymer with the thermoplastic polymer or the functional moiety may form short branches on the thermoplastic polymer.

In addition or as an alternative, embodiments of the thermoplastic-based polymer adhesive may contain other components. For example, thermoplastic-based polymer adhesive may contain fillers, such as, particles and fibers of organic and/or inorganic nature, for instance, particles and/or fibers of polymers, glass, ceramics (including bioceramics), and/or carbon. These fillers may be inert or have limited reactivity with the thermoplastic polymer of the adhesive.

Yet other components may include a filler material which absorbs the specified activation energy. Specific absorbents may include microwave energy absorbents to facilitate heating of the adhesive when it is exposed to microwave energy. The filler may include a conductive micro-scale or nano-scale filler. Additionally, it is possible to include an organic semiconductor, such as, a polypyrrole, a polyanilin, a polyacetylin, and their derivatives or a mixture thereof in the adhesive. Alternatively, the thermoplastic polymer of the adhesive may be a conductive polymer, such as, one or more copolymers of the lactides and pyrrole. The adhesive may then heat during application of the electrical current either by conduction from the heated conductive filler or directly by the electrical current. Still other components may include those that react with changes in pH or the ionic environment to cause the adhesive to change chemically.

The thermoplastic-based polymer adhesive may in addition or alternatively contain therapeutic agents including, but not limited to, antibacterial agents, fluoride releasing agents, and anti-caries agents. It will be appreciated that there may be other suitable fillers and agents and that embodiments of the invention are not limited to any particular therapeutic agent.

In this regard, the chemical changes produced by activation of the adhesive affect the properties of the adhesive. The chemical changes may include one or more improvements in the bond strength with a particular tooth material, improvements in creep resistance, improvements in the rigidity, and changes in the viscosity response to a change in temperature relative to the thermoplastic polymer alone. It will be appreciated that the desired chemical change may facilitate targeted property improvements for specific applications of the adhesive. For example, where polishability of the adhesive is required, the additional polymers or additives, once activated, may endow the adhesive with vastly improved polishability over that of the thermoplastic polymer or an organic/inorganic hybrid alone. Thus, prior to activation, the adhesive exhibits reversibility as to the viscosity, but once activated, the adhesive may not exhibit reversible viscosity properties. Instead, the adhesive may become polishable to an extent that exceeds that which is obtainable with the thermoplastic polymer alone. In restorative applications, for example, the reacted thermoplastic-based polymer adhesive may be used in applications where aesthetic surface finishes are required. It will be appreciated that multiple improvements may be observed in the adhesive for any particular application.

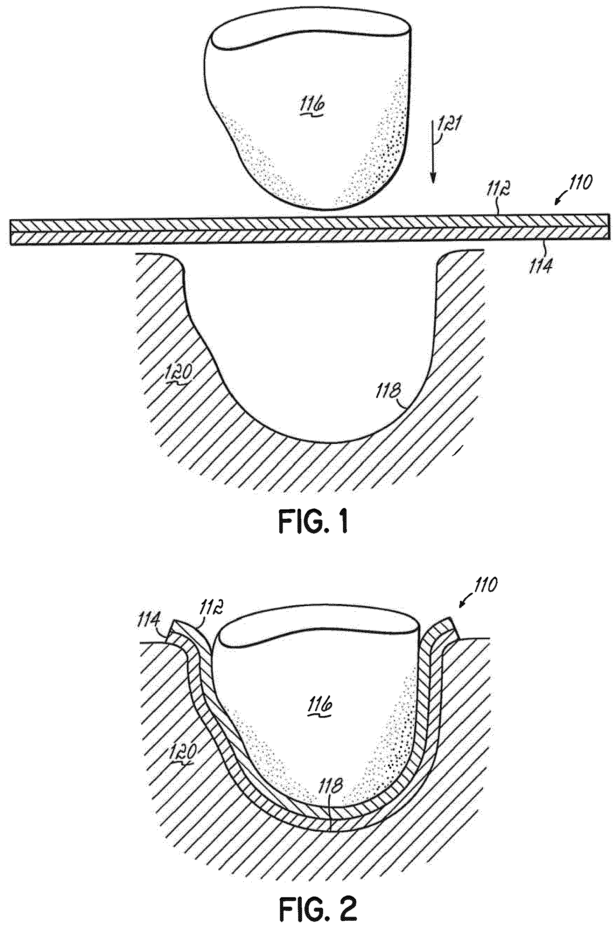

In one exemplary embodiment, not shown and not in accord with the invention, the thermoplastic-based polymer adhesive may be in the form of a single, homogenous layer, coating, sheet, or rod. It will be appreciated, however, that the layer may contain multiple different layers of adhesives. For example, as shown in Figs. 1 and 2, a layered sheet 110 may include a first layer 112 of a first thermoplastic-based polymer adhesive in direct contact or bonded to a second layer 114 of a second thermoplastic-based polymer adhesive. The first and second layers 112, 114 may be made of or contain different thermoplastic-based polymer adhesives. In this regard, the thermoplastic polymer of the first adhesive and the thermoplastic polymer of the second adhesive may have different molecular weights, have different adhesion characteristics, have other different chemical and/or physical properties, and/or have different filler loadings, to name only a few of the possible differences.

Furthermore, one or both of the layers 112, 114 may contain one or more of the additional polymers or additives, such as, the moieties, monomers, and/or prepolymers set out above. In this regard, each layer 112, 114 may be configured to bond to a particular surface. That is the additional polymers or additives are selected to enhance bonding with a predetermined surface. Additionally, only one of the layers 112, 114 may be activated during installation of the dental component. It will be appreciated, however, that both layers 112, 114 may be selectively activated at different times or simultaneously.

The sheet 110 may be positioned between a restoration 116 and an exemplary cavity 118 prepared in a tooth 120. Accordingly, the first layer 112 may be configured to bond to the surface of the restoration 116 while the second layer 114 is configured to bond to the dentin and/or the enamel of the tooth 120. Upon heating of the adhesive of the first layer 112 and/or the second layer 114 of the sheet 110, such as by application of ultrasonic energy, the restoration 116 may be seated or installed (as indicated by arrow 121) in the cavity 118. Application of energy may be simultaneous with the installation process. The bonding between the restoration 116 and the cavity 118 prior to activation may be reversed by application of energy as set forth herein.

Once it is determined that the restoration 116 is properly positioned, ultrasonic energy or another activation energy may be used to activate one or both of the adhesives in the layers 112 and 114. That is, where the reversibility of the respective adhesives is no longer needed or desired, application of the activation energy to activate one of the additional polymers or additives set forth above may result in a more permanent bond between the restoration 116 and the tooth 120. As a result of the structure of the layered sheet 110, the bond strength, as well as other desired characteristics of the bonds between the layers 112 and 114 and the corresponding surfaces, may be tailored upon activation. In addition to bond strength improvement, other properties may also be improved depending on the composition of the adhesive.

Where additional polymers or additives are to be mixed into the thermoplastic polymer, the additional polymers or additives may be encased within a plurality of capsules or other hollow containers and then be mixed with the thermoplastic polymer. This may be advantageous where the additional polymers or additives react spontaneously with the thermoplastic polymer. In this way, the additional polymers or additives may be separated from the thermoplastic polymer until needed or required.

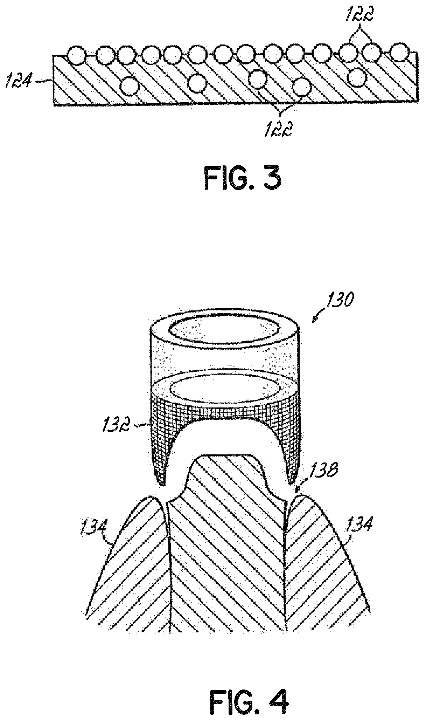

By way of example, as is shown in Fig. 3, in another embodiment of the invention, the thermoplastic-based polymer adhesive may be impregnated with a plurality of capsules 122 containing one or more additional polymers or additives that are reactive with the thermoplastic polymer or that can react and polymerize separately either through a self-curing mechanism or via light or heat curing mechanism. In one embodiment, the ingredients may include one or more of the additional polymers or additives set forth above.

The capsules 122 may be hollow spherical shells or other hollow non-spherical shell configurations with the additional polymers or additives, i.e., the core, contained within the surrounding shell. The shell material may have the same composition as the thermoplastic polymer or the shell material may be made of another material, such as, a brittle material, that shatters, ruptures, or melts upon exposure to the activation energy. The activation energy may induce pores in the shell or otherwise degrade the shell material to release the additional polymer or additive from the capsule 122. In this regard, the capsules 122 may also contain an ingredient which facilitates rupture of the capsule 122 when exposed to the activation energy of a predetermined magnitude or frequency or other threshold level. In one embodiment, the capsules 122 are ruptured by application of ultrasonic energy of a specific frequency or power level.

The capsules 122 may be micro-scale or nano-scale capsules that may be included in a layer 124 or in another layer for use in dentistry or dental related applications. Therefore, the capsules 122 may be included in one or both layers 112, 114 of the sheet 110, described above. The capsules 122 may be present in sufficient concentration so as to behave as a mechanically reinforcing phase. This reinforcement function may be achieved with or without any changes to the reversible property (for example, a reversible viscosity-temperature relationship) of the thermoplastic-based polymer adhesive.

Depending on the additional polymer or additive contained within the capsules 122, the concentration of the capsules 122 may range from about 0.1 wt.% to about 60 wt.% of the thermoplastic-based polymer adhesive. While reference is made to capsules 122 having the same ingredient contained therein, it will be appreciated that another set or multiple sets of capsules (not shown) each containing different reactive ingredients may be dispersed in the layer 124 such that, upon rupture, different reactive ingredients are released in the layer 124. Further, the different capsules may be activated under different conditions or under different activation energies or under different activation energy levels. The clinician may then time the release of the various contents from their respective capsules.

Once activated, the thermoplastic-based polymer adhesive may thus have additional properties that are more associated with the resin-based polymer chemistry. The layer 124 may be characterized by a blend of thermoplastic and thermosetting properties. For instance, the activated thermoplastic-based polymer adhesive may have improved polishability relative to the bulk thermoplastic polymer alone or the organic/inorganic hybrid phase.

With continued reference to Fig. 3, the capsules 122 may be disposed at the surface of the layer 124. Rupture of the capsules 122 at this location may change the properties in a region including the surface, as set forth above. In addition, in applications in which layer 124 is brought into contact with a specific material, the capsules 122 may contain an ingredient which improves the bonding with that specific material. For example, where the layer 124 is to be bonded with the dentin phase of a tooth, the capsules 122 may contain an ingredient that is capable of diffusing into the tubular pore structure of the dentin phase while also reacting with the thermoplastic polymer of layer 124. So, where the thermoplastic polymer of the adhesive may not be capable of penetrating into the pores of the dentin phase, the capsules 122 may be used to transport an ingredient that has this capability to the interface between the layer 124 and the dentin.

As set forth above, at the appropriate time, the reactive ingredient may be selectively released. By way of example, this may include during installation of a dental component as described above. While the capsules 122 are shown at the surface of the layer 124, embodiments of the invention are not limited to the configuration shown. It will be appreciated that the capsules 122 may, alternatively or in addition thereto, be dispersed throughout the layer 124, such that the rupture of the capsules 122 may release the additional polymer or additive contained therein to react substantially with the entire layer 124. Alternatively, even where the capsules 122 are dispersed throughout the layer 124, the additional polymer or additive may be preferentially disposed to migrate or diffuse to the surfaces of the layer 124 and react at those surfaces. For example, the capsules 122 may contain a highly hydrophobic resin which may be capable of migrating to the surface of the layer 124 to enhance one or more properties of the surface, such as, polishability. In addition, the capsules 122 may contain one or more moieties that can diffuse inside the tubules present within the dentin of a tooth to further enhance the physical/chemical anchoring properties.

As is known, gingival retraction may be necessary prior to taking a dental impression and may be one step in the preparation of a crown or a bridge in dental treatment. Typically, to retract the gingiva, a clinician packs a cord of material in the gingival sulcus space about the tooth. The clinician later removes the cord, which leaves an enlarged space surrounding the tooth. This procedure is difficult, tedious, and usually results in trauma and/or bleeding of the gingiva.

In accord with of the invention, one or more of the above-identified thermoplastic-based polymer adhesives may be used in a gingival retraction procedure. By way of example, the thermoplastic-based polymer adhesive may include polyamide, polycarbonate, and/or acrylonitrile-butadiene-styrene. The thermoplastic polymer composition may be in the form of a rod (not shown and not in accord with the invention) having a diameter of about 1 mm. A sonic or ultrasonic source may be brought into contact with the rod. Energy from the source may be used to soften the thermoplastic polymer at the tip. Once softened, the tip of the rod may be inserted into the gingival sulcus. This process may be repeated until the gingival sulcus is filled with the thermoplastic polymer composition. After some time, typically on the order of about 5 minutes, the thermoplastic polymer composition may be removed from the gingival sulcus with a dental instrument so as to leave the sulcus with an enlarged opening (not shown).

In one embodiment, the thermoplastic-based polymer adhesive, as described herein, is formed into an annular configuration or ring 130, shown in Fig. 4. The ring 130 may be attached to a sonic or an ultrasonic energy source. During application, the energy from the source softens a lower portion 132 of the ring 130 so that it is in a deformable state. While in the deformable state, the lower portion 132 of the ring 130 may be inserted into the sulcus 138 between the gum 134 and the tooth 136. The deformed ring is later (about 5 minutes later, after cooling sufficiently to harden) removed with a dental instrument and the sulcus 138 is left with an opening (not shown).

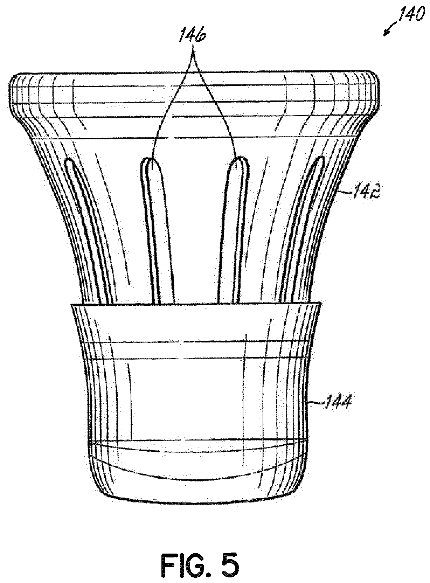

In another embodiment and with reference to Fig. 5, a cap-shaped device 140 includes an upper portion 142 and a lower portion 144. Each of the upper portion 142 and the lower portion 144 define a tubular-shaped cavity configured to fit over a tooth (not shown) for gingival retraction. The device 140 may be made of rubber. However, the upper portion 142 may be generally more rigid than the lower portion 144. In one embodiment, a plurality of separate, hollow channels 146 extend from the upper portion 142 to the lower portion 144. The hollow channels 146 may be filled with one or more of the thermoplastic-based polymer adhesives described herein.

During a retraction procedure, the device 140 may be positioned over a tooth where the lower portion 144 may be pushed into contact with the gingiva. A sonic or ultrasonic source is brought into contact with the upper portion 142 of the device 140. Application of energy through the upper portion 142 may cause the thermoplastic polymer to soften whereby it may be pushed from the hollow channels 146 into the gingival sulcus to retract the gum from the tooth. The retraction procedure may be continued until the thermoplastic-based polymer adhesive in all of the channels 146 is pushed into the sulcus. After about 5 minutes, the thermoplastic-based polymer adhesive is removed from the sulcus, leaving an enlarged opening (not shown).

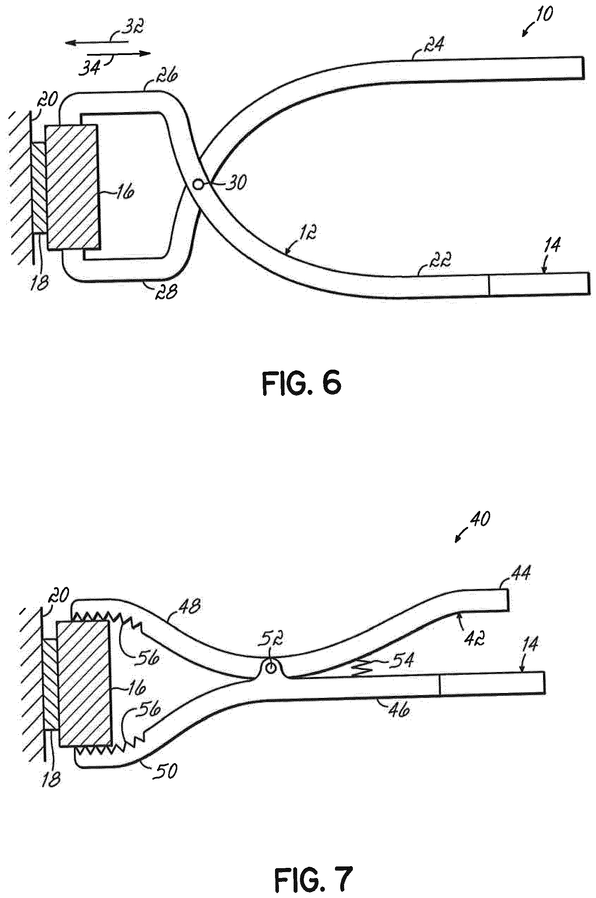

Figs. 6 and 7show a device 10 that includes a clamping portion 12 and a transducer portion 14 in operative communication therewith, each described in detail below. The device 10 may be used by the clinician to remove a dental component 16, for example, an orthodontic bracket, that is secured with a layer 18 of the thermoplastic-based polymer adhesive to a surface 20. The surface 20 to which the layer 18 is bonded may be defined by the enamel or the dentin phase of a tooth and may depend on the type of component 16 or application for which the component 16 is to be used. For example, where the component 16 is an orthodontic bracket, the surface 20 is typically the enamel portion of the tooth. Once removed, the component 16 may then be reattached to the surface 20 as disclosed herein.

With reference to Fig. 6, the device 10 may be sized to fit in a human hand. As such, the clamping portion 12 may include opposing handles 22, 24 with corresponding jaws 26, 28 that pivot about a joint 30. Squeezing the handles 22, 24 toward one another may therefore produce a similar movement of the jaws 26, 28. The jaws 26, 28 may be positioned in contact on opposing sides of the component 16. By a squeezing motion on the handles 22, 24, compressive pressure is applied to the component 16. In addition, the contact between one or both of the jaws 26, 28 produces a continuous mechanical pathway from the transducer portion 14 to the layer 18 through the component 16.

In this regard, the transducer portion 14 may be operatively coupled to the handle 22, as shown. The transducer portion 14 may include a sonotrode and/or an ultrasonic transducer capable of directing or generating sonic and/or ultrasonic vibrations and transmitting those vibrations to the clamping portion 12. By way of example, the transducer portion 14 may be capable of producing sonic or ultrasonic energy having a frequency in the range of from about 2 kHz to about 200 kHz, and by way of further example, the frequency may be in the range of from about 20 kHz to about 80 kHz. The ultrasonic energy may have oscillation amplitude of from about 1 µm to about 200 µm with a power of from about 2 to about 20 W/mm2 of targeted surface.

While the transducer portion 14 is shown as being secured or coupled to the handle 22, it will be appreciated that the transducer portion 14 may be located on the other handle 24 or on one or both jaws 26, 28. Other positions and orientations may be desirable and embodiments of the present invention are not limited to the relative positions of the transducer portion 14 and the clamping portion 12 shown in the figures. In this regard, the location of the transducer portion 14 is not limited as long as transmission of a sufficient amount of ultrasonic energy to the layer 18 selectively occurs as is described below.

With reference to Fig. 6, the device 10 may be used to remove the component 16 previously secured to the tooth 20 via layer 18. In this regard, the jaws 26, 28 may be brought into contact with opposing sides of the component 16. Application of pressure to the component 16 via the jaws 26, 28 produces frictional contact between the device 10 and the component 16. Ultrasonic energy may be selectively produced and passed from the transducer portion 14 through one or both jaws 26, 28, through the frictional contact between the clamping portion 12 and the component 16, through the component 16, and into the layer 18. Removing the component 16 may include forcibly pushing the component 16 via the frictional contact between the jaws 26, 28 toward the tooth 20 (as shown by arrow 32) thereby placing at least a portion of the layer 18 in compression. The transmitted ultrasonic energy may locally heat the thermoplastic-based polymer adhesive of the layer 18 to a sufficient temperature to reduce the viscosity or soften the thermoplastic polymer without activating the adhesive, as set forth above.

Once at least the thermoplastic polymer of the layer 18 is softened, the component 16 may be pulled in the direction of arrow 34 in Fig. 6 to debond or detach the component 16 from the tooth 20. Tensile stresses produced by pulling on the component 16 with the device 10 may be simultaneous with application of the ultrasonic energy to the layer 18. The combination of tensile stress and ultrasonic energy may facilitate detachment of the component 16 from the surface 20. It will be appreciated that detachment ideally occurs at the interface between the layer 18 and the surface 20 but other separation locations are possible. In this regard, it will also be appreciated that there may be applications in which it is desirable to have the layer 18 separate entirely within the layer 18 itself. In this instance, the portions of the layer 18 may reside on the detached component 16 and on the tooth 20. Alternatively, the layer 18 may be configured to detach at the interface between the component 16 and the layer 18. In such case, the layer 18 would then remain on the surface 20. However, in each instance, the device 10 advantageously allows the clinician to push to apply compressive stresses and then pull or apply tensile stresses to the layer 18 while selectively exposing the layer 18 to ultrasonic energy.

In this manner, the component 16 is undamaged during removal. Furthermore, where the layer 18 remains on the component 16, the component 16 may be reattached to the surface 20 with the device 10 at another location by application of ultrasonic energy while the layer 18 is compressed against the tooth 20. Advantageously, the device 10 may therefore allow a clinician to adjust the placement of the component 16 on the tooth 20 without degradation of the bond strength of the layer 18. This may entail multiple adjustments to the position of the component 16 by attaching and detaching the component 16 according to the above process.

Once the correct position for the component 16 is achieved, the device 10 may be used to activate the thermoplastic-based polymer adhesive, as set forth above, to more permanently bond the component 16 to the tooth or to cause a chemical reaction so as to improve one or more other properties of the layer 18 as set forth above. At this point, the reversibility of the viscosity-temperature relationship of the thermoplastic-based polymer adhesive may be reduced or eliminated.

With reference to Fig. 7 in which like reference numerals refer to like elements of Fig. 6, a device 40 may provide a similar function as that of the device 10 set out above. Specifically, for example, the device 40 may be configured to apply tensile or decompression forces to the layer 18 while directing ultrasonic energy to the layer 18. To this end, the device 40 may be a hand-held device and may include a clamping portion 42 and the transducer portion 14. The clamping portion 42 may include opposing handles 44, 46 and corresponding opposing jaws 48, 50. The handles 44, 46 and jaws 48, 50 may be operatively connected at joint 52. A spring 54 may be disposed between the handles 44, 46. By the configuration shown, the spring 54 may continuously force the jaws 48, 50 together. As shown, the jaws 48, 50 may include serrations or teeth 56 to facilitate frictional contact between the component 16 and the device 40.

In operation, the clinician may compress the handles 44, 46 toward one another to compress the spring 54. Once the jaws 48, 50 are positioned relative to opposing sides or portions of the component 16, the clinician may release the handles 44, 46 to allow the jaws 48, 50 to frictionally engage the component 16 under the load of the spring 54.

In a similar manner as set forth above with respect to the device 10, the device 40 may selectively apply both compressive and tensile stresses on the layer 18 while simultaneously transmitting or exposing the layer 18 to the ultrasonic energy from the transducer portion 14. Advantageously, this combination of tensile and ultrasonic energy allows the component 16 to be removed or detached from the tooth 20. As set forth above, the device 40 may be used to attach or reattach the component 16 to the tooth 20. Furthermore, the device 40 may be used to selectively activate the thermoplastic-based polymer adhesive of the layer 18.

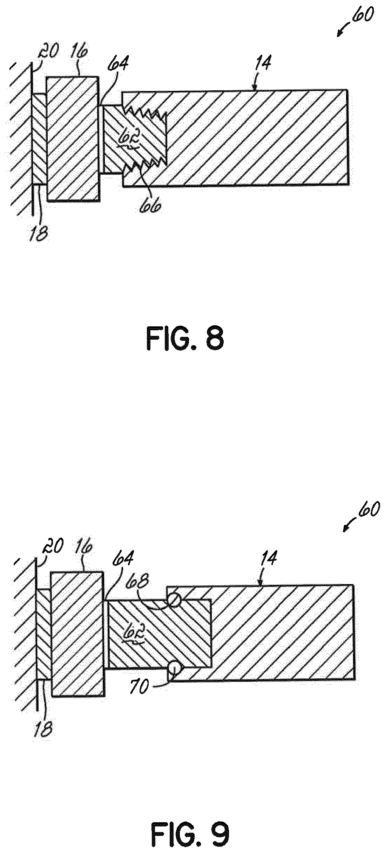

Fig. 8 in which like numerals refer to like elements of Figs. 6 and 7 shows a device 60 for removing and/or reattaching the component 16 from/to the tooth 20. To this end, the device 60 may include the transducer portion 14 and a linking member 62 with a layer 64 including the thermoplastic-based polymer adhesive thereon. The transducer portion 14 is operatively coupled to the linking member 62. By way of example, the transducer portion 14 may be threaded via thread 66 onto the linking member 62 having a corresponding thread. The transducer portion 14 may be threaded onto the linking member 62 prior to or after the linking member 62 is brought into contact with the tooth 20. By this connection between the transducer portion 14 and the linking member 62, ultrasonic energy may pass to the linking member 62.

As shown in Fig. 8, the adhesive layer 64 opposes the transducer portion 14 at one end of the linking member 62. The thermoplastic-based polymer adhesive of the layer 64 differs from the thermoplastic-based polymer adhesive of the layer 18. The difference between the compositions facilitates removal and placement of the component 16 with the device 60.

Specifically, in operation, the linking member 62 may be secured to the transducer portion 14 via thread 66. The adhesive layer 64 may then be brought into contact with the component 16 that is bonded to the tooth 20. The transducer portion 14 may be activated to produce a first ultrasonic energy, for example, at a first frequency and/or amplitude. By such first ultrasonic energy, the thermoplastic-based polymer adhesive of layer 64 is heated and softens as a result, though the adhesive is not activated. In one embodiment, the first sonic or ultrasonic energy is insufficient to activate the thermoplastic-based polymer adhesive of the layer 18. Following heating, the transducer portion 14 is turned off and the adhesive layer 64 is allowed to cool. Upon cooling, the linking member 62 is secured to the component 16 via the adhesive layer 64.

To remove the component 16 from the tooth 20, the transducer portion 14 may be again energized. However, the transducer portion 14 is modified so as to produce a second ultrasonic energy different than the first ultrasonic energy by frequency and/or amplitude. The second ultrasonic energy is tailored to the thermoplastic-based polymer adhesive of the layer 18 and not the thermoplastic-based polymer adhesive of the adhesive layer 64. The adhesive layer 64 may transmit all or a majority of the second ultrasonic energy to the layer 18 which, as set forth above, causes the layer 18 to heat. The adhesive layer 64 may not heat sufficiently such that it does not soften appreciably. In one embodiment, the second sonic or ultrasonic energy is insufficient to activate the thermoplastic-based polymer adhesive of adhesive layer 64. Once the layer 18 is sufficiently heated and softened, the component 16 may be pulled from the tooth 20 thereby detaching the component 16 from the tooth 20.

During the detachment process, the device 60 may be pushed and pulled to apply compressive and then tensile stresses, respectively, on the layer 18 as set forth above. Specifically, for example, the device 60 may be initially pushed toward the tooth 20 while the transducer portion 14 is activated to produce the second ultrasonic energy. In one embodiment, the first sonic or ultrasonic energy differs in frequency from the second sonic or ultrasonic energy. In one embodiment, the first sonic or ultrasonic energy differs in amplitude from the second sonic or ultrasonic energy.

Once the layer 18 is heated sufficiently, the device 60 may be pulled so as to place the layer 18 under tension to cause it to separate from the tooth 20 while in a softened state. In one embodiment, the thermoplastic polymer of the layer 18 has a lower softening temperature than the thermoplastic polymer of the adhesive layer 64. The component 16, once detached, may be reattached to the tooth 20 by reversing the order in which the first and second ultrasonic energies are applied to the adhesive layer 64 and the layer 18, respectively. Once reattached to the tooth 20, the layer 18 may be activated, as set forth above, to provide a more permanent bond between the tooth 20 and the component 16 or to improve some other attribute of the layer 18.

In an alternative device to that shown in Fig. 8, the linking member 62 shown in Fig. 9 includes a notch or depression 68 which may be configured to receive a snap-lock mechanism 70 of the transducer portion 14. It will be appreciated that the reverse construction of the depression 68 and snap-lock mechanism 70 is also contemplated. Operation of the snap-lock mechanism 70 in cooperation with the depression 68 allows for quick, convenient mechanical connection between the linking member 62 and the transducer portion 14. Thus, during treatment, when it becomes necessary to quickly debond and reattach multiple components 16, such as brackets, and then activate the thermoplastic-based polymer adhesive, the clinician may save substantial time through the use of the devices.

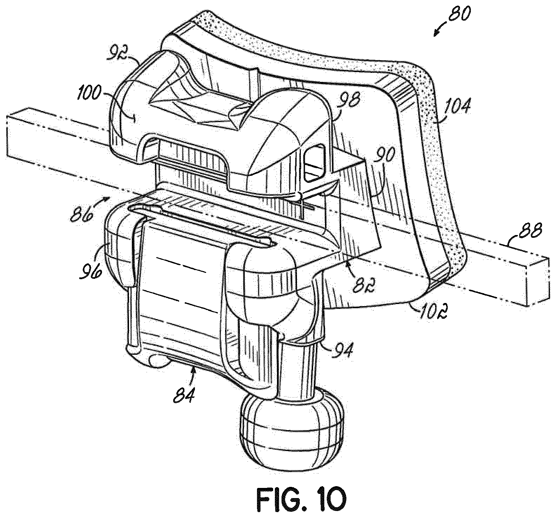

The thermoplastic-based polymer adhesive, as set forth herein, is configured to be used in conjunction with bonding an orthodontic bracket to a tooth, as briefly described above. Orthodontic brackets are known in the art and include orthodontic brackets disclosed in U.S. Patent No. 8,033,824. One exemplary orthodontic bracket is shown in Fig. 10.

With reference to Fig. 10, the orthodontic bracket 80 includes a bracket body 82 and a ligating slide 84 slidably coupled with the bracket body 82. The bracket body 82 includes an archwire slot 86 formed therein adapted to receive an archwire 88 (shown in phantom) for applying corrective forces to the teeth. When mounted to the surface of a tooth carried on the patient's lower jaw, the bracket body 82 has a lingual side 90, an occlusal side 92, a gingival side 94, a mesial side 96, a distal side 98, and a labial side 100. In one embodiment, the lingual side 90 of the bracket body 82 may be configured to be secured to the tooth with a thermoplastic polymer composition disclosed herein. It will be appreciated that the lingual side 90 of the bracket 80 may further be provided with a pad 102 defining a bonding base that is configured to be secured to the surface of the tooth 20 (labeled in Fig. 6). In this instance, a thermoplastic-based polymer adhesive as disclosed herein may be in the form of a coating positioned between the pad 102 and the tooth 20 and which is configured to rigidly bond the bracket 80 thereto for orthodontic treatment. While the orthodontic bracket 80 is shown to include a ligating slide 84, it is known that orthodontic brackets need not include a ligating member.

As shown, the thermoplastic-based polymer adhesive may be in the form of a coating 104 that is placed on the pad 102. By way of example, the coating 104 may be formed by spraying the thermoplastic-based polymer adhesive onto the pad 102 prior to installation, such as, during the manufacturing process for the orthodontic bracket 80. Thus, the coating 104 may be factory applied. Advantageously, this configuration eliminates the need for the clinician to apply any cement to the tooth or bracket prior to installing the bracket 80 thereby simplifying installation of brackets to correct the patient's malocclusion.

The clinician may alternatively apply the coating 104 to the pad 102 or bracket 80 as desired. For example, other methods may include dipping or brushing a layer of material that contains the thermoplastic-based polymer adhesive onto the pad 102. In yet another alternative, the thermoplastic-based polymer adhesive may be in the form of a separate sheet or a rod (not shown), which may be sonically or ultrasonically welded to the pad 102 prior to installation of the bracket 80 on a tooth or the sheet or rod may be sandwiched between the pad 102 and the tooth 20 during installation of the bracket 80 on a patient's tooth. The bond between the tooth 20, the thermoplastic-based polymer adhesive, and the pad 102 may then be formed substantially simultaneously according to one of the methods disclosed herein.

As an alternative to application of ultrasonic energy or other energy source, described above, the bond between one or both of the thermoplastic-based polymer adhesive and the pad 102 and/or the tooth 20 may be achieved by passing an electrical current through the adhesive at a power sufficient to heat the polymer. Heat may then be a result of resistive heating, i.e., joule heating. In general, electrical current may flow through the polymer itself or through a conductive filler, as set forth above, mixed into the thermoplastic polymer. In any respect, current flow may cause the adhesive to soften.

Once sufficiently softened, the thermoplastic-based polymer adhesive may be bonded to the tooth 20 and/or the bracket 80. Typically, this is achieved by compressing the softened composition between the pad 102 of the bracket 80 and a tooth. Upon cooling of the softened composition, the bracket 80 becomes sufficiently rigidly bonded to the tooth for orthodontic treatment. As set forth above, the heating and softening properties of the thermoplastic-based polymer adhesive may be reversible, prior to activation, such that the bracket 80 may be removed and reattached as necessary to accurately position the bracket 80 on the tooth 20. Thus, use of the thermoplastic-base polymer adhesive, whether as a coating preapplied to the bracket 80 or applied by the clinician in sheet or rod form, may allow the clinician to reversibly attach and detach the bracket 80 more than one time should the initial placement of the bracket 80 be inaccurate. However, once the bracket 80 is properly positioned, the thermoplastic-based polymer adhesive may be activated as set out above. Such activation may result in improved bond strength, or improvement in another desired attribute. However, the reversibility of the viscosity with temperature may be eliminated or significantly reduced.

While the present invention has been illustrated by a description of various embodiments and while these embodiments have been described in some detail, additional advantages and modifications will readily appear to those of ordinary skill in the art.

## Claims

1. A device for use in a gingival retraction procedure to enlarge a gingival sulcus, the device **characterised in that** it comprises: a thermoplastic-based polymer adhesive comprising a thermoplastic polymer, and an additional polymer or additive including at least one moiety, monomer, or prepolymer capable of changing at least one property of the thermoplastic-based polymer adhesive when exposed to an activation energy, and a tubular wall or ring structure (130), (140) configured to fit around a tooth and including the thermoplastic-based polymer adhesive, the structure (130), (140) being configured to be operatively coupled to a source of energy that is capable of applying energy to the thermoplastic-based polymer adhesive to heat the thermoplastic-based polymer adhesive before the thermoplastic-based polymer adhesive is inserted into the sulcus.

2. The device of claim 1 wherein the tubular wall or ring structure (140) defines channels (146) that contain the thermoplastic-based polymer adhesive, the channels (146) being configured to be in communication with a gingiva when the structure (140) is placed around the tooth, wherein the thermoplastic-based polymer adhesive is movable within the channels (146) toward the sulcus.

3. The device of claim 1 wherein a portion of the tubular wall or ring structure (130) that is configured for contact with a gingiva is formed of the thermoplastic-based polymer adhesive.

4. The device of any preceding claim wherein the activation energy is heat energy and the additional polymer or additive is activated at a predetermined temperature.

5. The device of any one of claims 1 to 3 wherein the activation energy is sonic or ultrasonic energy.

6. The device of any preceding claim wherein the additional polymer or additive is contained within a capsule that is configured to release the additional polymer or additive upon application of the activation energy.

7. The device of any preceding claim wherein the additional polymer or additive is capable of modifying or reacting with the thermoplastic polymer to modify at least one of the bond strength and the polishability of the thermoplastic polymer.

8. The device of any preceding claim wherein the additional polymer or additive includes a moiety capable of diffusing into the tubular structures of the dentin phase of a tooth.

9. The device of any preceding claim wherein the thermoplastic polymer is selected from the group consisting of polyamides, polyaryl ketones, polyesters, polyimides, polysulfones, polyurethanes, polycarbonates, polyolefines, halogenated polyolefines, polyacetals, polyacrylates, polymethacrylates, and polyphenylene sulfides.

10. The device of any preceding claim wherein the additional polymer or additive is bisphenol A glycidyl methacrylate (BisGMA), urethane dimethacrylate, triethylene glycol dimethacrylate, or a derivative thereof.

## Patentansprüche

1. Vorrichtung zur Verwendung in einem Zahnfleischretraktionsverfahren zum Vergrößern einer Zahnfleischtasche, wobei die Vorrichtung **dadurch gekennzeichnet ist, dass** sie Folgendes umfasst: einen Polymerklebstoff auf Thermoplast-Basis, der ein thermoplastisches Polymer umfasst, und ein zusätzliches Polymer oder Additiv mit wenigstens einem Molekülteil, Monomer oder Vorpolymer, das, wenn es einer Aktivierungsenergie ausgesetzt wird, wenigstens eine Eigenschaft des Polymerklebstoffs auf Thermoplast-Basis ändern kann, und eine tubuläre Wand- oder Ringstruktur (130), (140), so konfiguriert, dass sie um einen Zahn passt und den Polymerklebstoff auf Thermoplast-Basis beinhaltet, wobei die Struktur (130), (140) zum operativen Koppeln mit einer Energiequelle konfiguriert ist, die Energie auf den Polymerklebstoff auf Thermoplast-Basis applizieren kann, um den Polymerklebstoff auf Thermoplast-Basis vor dem Einführen des Polymerklebstoffs auf Thermoplast-Basis in die Tasche zu erhitzen.

2. Vorrichtung nach Anspruch 1, wobei die tubuläre Wand- oder Ringstruktur (140) Kanäle (146) definiert, die den Polymerklebstoff auf Thermoplast-Basis enthalten, wobei die Kanäle (146) so konfiguriert sind, dass sie mit einem Zahnfleisch in Verbindung sind, wenn die Struktur (140) um den Zahn gelegt wird, wobei der Polymerklebstoff auf Thermoplast-Basis in den Kanälen (146) in Richtung der Tasche beweglich ist.

3. Vorrichtung nach Anspruch 1, wobei ein Teil der tubulären Wand- oder Ringstruktur (130), der für einen Kontakt mit einem Zahnfleisch konfiguriert ist, aus dem Polymerklebstoff auf Thermoplast-Basis gebildet ist.

4. Vorrichtung nach einem vorherigen Anspruch, wobei die Aktivierungsenergie Wärmeenergie ist und das zusätzliche Polymer oder Additiv bei einer vorbestimmten Temperatur aktiviert wird.

5. Vorrichtung nach einem der Ansprüche 1 bis 3, wobei die Aktivierungsenergie Schall- oder Ultraschallenergie ist.

6. Vorrichtung nach einem vorherigen Anspruch, wobei das zusätzliche Polymer oder Additiv in einer Kapsel enthalten ist, die zum Freisetzen des zusätzlichen Polymers oder Additivs bei Applikation der Aktivierungsenergie konfiguriert ist.

7. Vorrichtung nach einem vorherigen Anspruch, wobei das zusätzliche Polymer oder Additiv das thermoplastische Polymer modifizieren oder damit reagieren kann, um die Haftfestigkeit und/oder die Polierbarkeit des thermoplastischen Polymers zu modifizieren.

8. Vorrichtung nach einem vorherigen Anspruch, wobei das zusätzliche Polymer oder Additiv einen Molekülteil enthält, der in die tubulären Strukturen der Dentinphase eines Zahns diffundieren kann.

9. Vorrichtung nach einem vorherigen Anspruch, wobei das thermoplastische Polymer ausgewählt ist aus der Gruppe bestehend aus Polyamiden, Polyarylketonen, Polyestern, Polyimiden, Polysulfonen, Polyurethanen, Polycarbonaten, Polyolefinen, halogenierten Polyolefinen, Polyacetalen, Polyacrylaten, Polymethacrylaten und Polyphenylensulfiden.

10. Vorrichtung nach einem vorherigen Anspruch, wobei das zusätzliche Polymer oder Additiv Bisphenol-A-Glycidylmethacrylat (BisGMA) Urethandimethacrylat, Triethylenglykoldimethacrylat oder ein Derivat davon ist.

## Revendications

1. Dispositif destiné à être utilisé dans une procédure de rétraction gingivale pour agrandir un sulcus gingival, le dispositif étant **caractérisé en ce qu'**il comprend : un adhésif polymère à base de thermoplastique comprenant un polymère thermoplastique, et un polymère supplémentaire ou additif comportant au moins une fraction, un monomère ou prépolymère capable de changer au moins une propriété de l'adhésif polymère à base de thermoplastique quand il est exposé à une énergie d'activation, et une structure de paroi ou d'anneau tubulaire (130), (140) configurée pour être ajustée autour d'une dent et comportant l'adhésif polymère à base de thermoplastique, la structure (130), (140) étant configurée pour être couplée fonctionnellement à une source d'énergie capable d'appliquer une énergie à l'adhésif polymère à base de thermoplastique afin de chauffer l'adhésif polymère à base de thermoplastique avant d'insérer l'adhésif polymère à base de thermoplastique dans le sulcus.

2. Dispositif selon la revendication 1 dans lequel la structure de paroi ou d'anneau tubulaire (140) définit des canaux (146) qui contiennent l'adhésif polymère à base de thermoplastique, les canaux (146) étant configurés pour être en communication avec une gencive quand la structure (140) est placée autour de la dent, dans lequel l'adhésif polymère à base de thermoplastique peut être déplacé à l'intérieur des canaux (146) vers le sulcus.

3. Dispositif selon la revendication 1 dans lequel une partie de la structure de paroi ou d'anneau tubulaire (130) qui est configurée pour faire contact avec une gencive est formée de l'adhésif polymère à base de thermoplastique.

4. Dispositif selon l'une quelconque des revendications précédentes dans lequel l'énergie d'activation est une énergie thermique et le polymère supplémentaire ou l'additif est activé à une température prédéterminée.

5. Dispositif selon l'une quelconque des revendications 1 à 3 dans lequel l'énergie d'activation est une énergie sonique ou ultrasonique.

6. Dispositif selon l'une quelconque des revendications précédentes dans lequel le polymère supplémentaire ou l'additif est contenu dans une capsule qui est configurée pour libérer le polymère supplémentaire ou l'additif à l'application de l'énergie d'activation.

7. Dispositif selon l'une quelconque des revendications précédentes dans lequel le polymère supplémentaire ou l'additif est capable de modifier ou de réagir avec le polymère thermoplastique pour modifier au moins l'une de la force d'adhésion et de l'aptitude au polissage du polymère thermoplastique.

8. Dispositif selon l'une quelconque des revendications précédentes dans lequel le polymère supplémentaire ou l'additif comporte une fraction capable de se diffuser à l'intérieur des structures tubulaires de la phase de dentine d'une dent.

9. Dispositif selon l'une quelconque des revendications précédentes dans lequel le polymère thermoplastique est sélectionné dans le groupe consistant en polyamides, polyarylcétones, polyesters, polyimides, polysulfones, polyuréthanes, polycarbonates, polyoléfines, polyoléfines halogénées, polyacétaux, polyacrylates, polyméthacrylates et polysulfures de phénylène.

10. Dispositif selon l'une quelconque des revendications précédentes dans lequel le polymère supplémentaire ou l'additif est le méthacrylate de glycidyle et de bisphénol A (BisGMA), le diméthacrylate d'uréthane, le diméthacrylate de triéthylèneglycol, ou un dérivé de ceux-ci.
